# EUROPEAN PATENT SPECIFICATION

(11) **EP 3 863 701 B1**
(45) Date of publication and mention of the grant of the patent: **10.04.2024**
(21) Application number: 19783049.0
(22) Date of filing: 09.10.2019
(51) Int. Cl.: A61M 5/32

(54) **AN INJECTION DEVICE**
INJEKTIONSVORRICHTUNG
DISPOSITIF D'INJECTION

(30) Priority: 12.10.2018 EP 18306346
(43) Date of publication of application: 18.08.2021
(73) Proprietor: SANOFI, 75008 Paris (FR)
(72) Inventor: DASBACH, Uwe, 65926 Frankfurt am Main (DE); ZUYEV, Alex, Bridgewater, New Jersey 08807 (US); KEMP, Thomas Mark, Cambridgeshire Melbourn Herts Cambridgeshire SG8 6DP (GB); REVELLAT, Hugo, Cambridgeshire Melbourn Herts Cambridgeshire SG8 6DP (GB); DENYER, Timothy, Cambridgeshire Melbourn Herts Cambridgeshire SG8 6DP (GB)
(74) Representative: Hudson, George Alec
(86) International application number: PCT/EP2019/077333
(87) International publication number: WO 2020/074570

(56) References cited:
- EP-A1- 2 175 917
- EP-B1- 2 175 917
- WO-A1-2005/070481
- US-B1- 7 771 397

## Description

### FIELD OF THE INVENTION

The present invention relates to an injection device with a removable cap and a method of assembling the cap of said injection device.

### BACKGROUND OF THE INVENTION

injection devices, such as auto-injectors, are known in the art for dispensing a medicament to the injection site of a patient. Such injection devices typically comprise a housing and a cap. A needle syringe is located in the housing and is sometimes covered by a needle shield. The cap and needle shield are removably attached to the housing to shield the needle of the needle syringe. To dispense the medicament, the cap and needle shield are first removed from the housing to expose the needle. The needle is then inserted into the body of the patient at the injection site to inject the medicament.

The force required to remove the cap from the housing can be relatively high which can be due to the frictional interface between the needle shield and the syringe. Infirm patients such as the elderly or physically impaired may find removing the cap difficult due to the relatively high forces required. Furthermore the force required to remove the cap can be increased further by cold temperatures, some medicaments and therefore some injection devices need to be stored in the fridge at low temperatures. This can exacerbate the difficulty in removing the cap from the housing.

US 7,771,397 B1 discloses a needle cover assembly comprising a manually operable member, a rigid needle shield provided with an internally arranged resilient cap, and a rigid needle shield remover assembly including a tubular shaped member surrounding the rigid needle shield. The tubular shaped member is arranged with at least one grip member capable of gripping the rigid needle shield in order to remove the rigid needle shield. The rigid needle shield remover assembly further includes a spinning attachment member having a distal end fixedly attached to the tubular shaped member and a proximal end rotatably attached to the manually operable member such that the manually operable member can be freely turned in relation to the rigid needle shield remover assembly but is locked in an axial direction.

WO 2005/070481 A1 discloses an injection device comprising an outer housing inside which is located a medicament-holding barrel with a needle at one end thereof, a plunger moveable within the barrel, an inner housing intermediate the outer housing and the barrel and plunger and an energy source in communication with said inner housing. The inner housing is moveable by the energy source between a first position in which the plunger and barrel are movable axially so as to move at least part of said needle out of the outer housing; a second position in which the plunger is movable axially into said barrel so as to expel medicament through the needle; and a third position in which the plunger and barrel are able to retract in order to retract the needle into the outer housing.

EP 2,175,917 A1 discloses an injection device comprising a locking mechanism between a syringe carrier and an end cap of the injection device. The cap inhibits movement of the syringe carrier and syringe towards an exit aperture of the injection device when the cap is located in its closed position on the injection device. A needle shield located on the needle of the syringe is removed by rotating the cap whilst the locking mechanism remains engaged. On removal of the cap, the locking mechanism is no longer so engaged and the syringe and syringe carrier can move towards the exit aperture. This assists in preventing damage to the syringe prior to actuation of the injection device.

### SUMMARY

It is an object of the present invention to provide an improved injection device having a removable cap.

According to the invention there is provided an injection device comprising an elongate housing having a central axis and a cap provided at a distal end of the housing, the cap being removable from the housing and comprising: an outer member for a user to grip when removing the cap from the housing; an intermediate member disposed substantially within the outer member, at least one of the intermediate member or outer member being rotatable relative to the other of the intermediate member or outer member; and, an inner member disposed substantially within the intermediate member and coupled to at least one of the outer member and the intermediate member, the inner member being configured to engage a needle shield of a syringe of medicament when such syringe is received in the housing, wherein the injection device further comprises a lifting mechanism configured to reduce a force required to remove the cap and an engaged needle shield from the housing, the lifting mechanism comprising at least one axially-inclined portion in engagement with at least one lifting portion such that rotational movement of the outer member or intermediate member is converted into an axial movement of the inner member in a distal direction from the housing.

According to the invention there is also provided an injection device comprising an elongate housing having a central axis and a cap provided at a distal end of the housing, the cap being removable from the housing and comprising: an outer member for a user to grip when removing the cap from the housing; an intermediate member disposed substantially within the outer member, at least one of the intermediate member or outer member being rotatable relative to the other of the intermediate member or outer member; an inner member disposed substantially within the intermediate member and coupled to at least one of the outer member and the intermediate member, the inner member being configured to engage a needle shield of a syringe of medicament when such syringe is received in the housing, and a lifting mechanism configured to reduce a force required to remove the cap and an engaged needle shield from the housing, the lifting mechanism comprising at least one axially-inclined portion in engagement with at least one lifting portion such that rotational movement of the outer member or intermediate member is converted into an axial movement of the inner member in a distal direction from the housing.

Advantageously the lifting mechanism can assist a user in removing the cap from the housing, therefore requiring less force from the user to remove the cap, relative to the force required without assistance. Infirm patients such as the elderly or physically impaired may find removing the cap difficult, so a reduction in the force required to remove the cap from the housing is beneficial. Furthermore if the injection device has been stored at low temperatures this can cause the required force to remove the cap from the housing to increase relative to the force required for removal of the cap at room temperature. This can cause further problems to those who already find removing the cap difficult.

The outer member can include a lid and the lid and the outer member can be separate components, the lid being fixed rotationally and axially to the outer member. However, it can be appreciated that the lid and the outer member could also be formed as a single component. Manufacturing the components as separate parts can enable a simpler assembly process, however manufacturing them as one component enables a reduction in the overall number of components in the device.

The outer member can include a lid and the inner member can comprise an aperture at a distal end. The aperture can be in engagement with a fixing portion on the lid such that the inner member is coupled to the lid so as to be fixed axially with respect to the lid and to be rotatable with respect to the lid. In addition or alternatively the outer member may comprise an annulus shaped portion which can be in engagement with clips on a distal end of the inner member such that the inner member is fixed axially with respect to the outer member and rotatable with respect to the outer member. Advantageously the inner member being rotatable to the outer member can mean that the needle shield is not rotated when the cap is removed. Rotation of the needle shield, while in situ covering the needle of a syringe, could damage the needle for example by coring.

The intermediate member and inner member may comprise a stop mechanism, the inner member is free to move axially in a distal direction with respect to the intermediate member until a first stop portion of the intermediate member engages with a second stop portion of the inner member such that the inner member is prevented from further axial movement relative to the intermediate member. The stop mechanism can prevent rotational movement of the inner member relative to the intermediate member. An advantage of this feature is that the intermediate portion is not axially fixed to the inner member until the stop portions are engaged, this enables the inner member to move the needle shield in a distal direction independently of the intermediate member, the components are then fixed axially for the final lift of the cap from the housing.

The axially-inclined portion can be a groove or cut out and the lifting portion can be a boss, the lifting portion is located substantially within the axially-inclined portion, wherein as the outer member is twisted the lifting portion is forced to follow a path of the axially-inclined portion, causing axial movement of the outer member and the inner member.

The axially-inclined portion can be a ridge or projection and the lifting portion can be a boss, the lifting portion is axially offset from the axially-inclined portion but in contact with a distal or proximal edge of the axially-inclined portion, wherein as the outer member is twisted the lifting portion is forced to follow a path of the axially-inclined portion causing axial movement of the outer member and the inner member in a distal direction.

An advantage of this is that a simple mechanism which is easy to manufacture and assemble enables a momentum build up to assist a user in removing a cap or needle shield from an injection device.

The axially inclined portion may be a first axially inclined portion located on one of an outer face of the intermediate member and an inner face of the outer member, and the lifting portion can be a first lifting portion on the other of the outer face of the intermediate member or the inner face of the outer member, the outer face being directed away from a central axis of the injection device and the inner face being directed towards a central axis of the injection device.

The lifting mechanism may comprise a second lifting portion and a second axially-inclined portion, the second lifting portion being located on one of an inner face of the intermediate member and an outer face of the inner member and the second axially-inclined portion being on the other of the outer face of the inner member and the inner face of the intermediate member, an outer face being directed away from a central axis of the injection device and an inner face being directed towards a central axis of the injection device. The second axially-inclined portion may have a shallower gradient with respect to the axial direction than the first axially-inclined portion.

Advantageously the intermediate member comprising two lifting portions enables a pulling force of the outer member to be converted into a twisting movement of the intermediate member and in turn an axial movement of the inner member to provide a mechanical advantage when removing the cap and the needle shield of the injection device. Furthermore the difference in gradients can enable a greater axial movement of the outer member to cause a smaller axial movement of the inner member relative to the axial movement of the outer member. The difference in gradients could also enable a smaller axial movement of the outer member to cause a larger axial movement of the inner member relative to the movement of the outer member if the first axially inclined portion has a shallower gradient with respect to the axial direction than the second axially inclined portion. Furthermore the axial force on the inner member can be reduced or increased relative to the axial force applied to the outer member based on the difference in gradients of the first and second axially inclined portions.

At least one of the axially inclined portions can be a groove or cut out and at least one of the lifting portions can be a boss, the at least one of the lifting portions is located substantially within the at least one of the axially-inclined portions, wherein as the outer member is lifted in a distal direction from the housing each lifting portion is forced to follow a path of each axially-inclined portion, causing rotational movement of the intermediate member and axial movement of the inner member.

At least one of the axially-inclined portions can be a ridge or projection and at least one of the lifting portions can be a boss, the at least one of the lifting portions is axially offset from the at least one of the axially-inclined portions but in contact with a distal or proximal edge of the at least one of the axially-inclined portions, wherein as the outer member is lifted in a distal direction away from the housing each lifting portion is forced to follow a path of each axially-inclined portion, causing rotational movement of the intermediate member and axial movement of the inner member.

An advantage of this is that a simple mechanism which is easy to manufacture and assemble enables a momentum build up to assist a user in removing a cap from an injection device.

The injection device may comprise a holding portion at one end of the axially-inclined portion or the second axially-inclined portion to engage with the lifting portion, wherein when the holding portion and the lifting portion are engaged the cap and the housing are held together and relative movement of the cap and the housing is resisted. It may be required that the cap is pushed in a proximal direction to release the lifting portion from the holding portion before removal of the cap. An advantage of this is that premature removal of the cap is prevented, this feature may also prevent movement of particular components of the device during storage or transport which could damage the device.

According to one aspect of the invention there is provided an injection device comprising: an elongate housing having a central axis and a cap provided at a distal end of the housing, the cap being removable from the housing and comprising: an outer member for a user to grip when removing the cap from the housing; and an inner member disposed substantially within the outer member, and coupled to the outer member such that the outer member is rotatable with respect to the inner member, the inner member being configured to engage a needle shield of a syringe of medicament when such a syringe is received in the housing; and a lifting mechanism configured to reduce a force required to remove the cap and an engaged needle shield from the housing, the lifting mechanism comprising: a lifting portion on one of the outer member and the housing, and an axially-inclined portion on the other of the outer member and the housing, wherein, the lifting portion engages with the axially-inclined portion such that rotational movement of the outer member with respect to the housing is converted into an axial movement of the inner member in a distal direction from the housing.

Advantageously the lifting mechanism can assist a user in removing the cap from the housing, therefore requiring less force from the user to remove the cap, relative to the force required without assistance. Infirm patients such as the elderly or physically impaired may find removing the cap difficult, so a reduction in the force required to remove the cap from the housing is beneficial. Furthermore if the injection device has been stored at low temperatures this can cause the required force to remove the cap from the housing to increase relative to the force required for removal of the cap at room temperature. This can cause further problems to those who already find removing the cap difficult.

The lifting portion can be a protrusion on the outer member extending axially in a proximal direction from a proximal edge of the outer member, the axially-inclined portion being on the housing. Alternatively the lifting portion can be a protrusion on the housing extending axially in a distal direction from a distal edge of the housing, the axially-inclined portion being on the outer member. Alternatively the lifting portion can be an inwardly protruding boss on an inner surface of the outer member, extending towards the central axis of the housing, the axially-inclined portion being on the housing. Alternatively the lifting portion can be an outwardly protruding boss on an outer surface of the housing, extending away from the central axis of the housing, the axially-inclined portion being on the outer member.

The axially-inclined portion can be a recessed portion for engagement with the lifting portion. Alternatively the axially-inclined portion can be a ridge or projection for engagement with the lifting portion. The axially-inclined portion can be curved. Alternatively the axially-inclined portion can be linear. Advantageously the axially inclined portion can be a number of varying profiles and gradients. These can be chosen based on the particular requirements of the injection device, for example but not limited to the required axial movement of the inner member, or the required force reduction in removing the cap for a user.

Before the cap is removed from the housing, the cap can be configured to engage with the housing such that the outer member extends in a distal direction over a proximal end of the housing to cover the lifting mechanism. This protects the lifting mechanism and provides a more aesthetic interface between the cap and the housing.

Before the cap is removed from the housing, the cap can be configured to engage with the housing such that the lifting mechanism is exposed. Advantageously this enables less material to be used in manufacturing the outer member.

The axially-inclined portion can comprise more than one gradient to provide varying levels of assistance in removal of the cap from the housing.

The housing and the outer member can meet at an interface, the housing and the outer member can be non-circular in cross section at this interface, perpendicular to the central axis of the housing. This enables more freedom in the cross-sectional design of the housing and the cap.

The injection device may comprise a syringe having a needle disposed within the housing, wherein said syringe contains a medicament.

The injection device can comprise a needle shield to cover the needle; furthermore the inner member may comprise an engagement member for engagement with the needle shield, wherein removal of the cap simultaneously removes the needle shield. The engagement member can be a grabber, a barb or deflectable arm or may be an alternative known way of fixedly coupling two components together. The engagement member may cut into the needle shield or clamp to the needle shield. The inner member being configured to engage the needle shield can mean that the inner member has a gripper or alternative as described above attached to the inner member, or has an integral part of the inner member to engage the needle shield. It shall be appreciated that infirm patients such as the elderly or physically impaired may find removing the needle shield more difficult than removing the cap due to the small size of the needle shield making it difficult to handle so coupling the two components together for simultaneous removal addresses this issue.

In some embodiments, the at least one axially-inclined portion is in engagement with the at least one lifting portion such that rotational movement of the outer member or intermediate member is converted into said axial movement of the inner member in the distal direction from the housing such that the axial movement of the inner member in the distal direction from the housing urges the engaged needle shield in the distal direction from the housing.

In some embodiments, the needle shield comprises a rigid outer member and a resilient inner member.

In some embodiments, the inner member is coupled to said at least one of the outer member and intermediate member and is configured to engage said at least one of the outer member and the intermediate member.

In some embodiments, the cap comprises the lifting mechanism.

In some embodiments, all of the inner, intermediate and outer members are removable from the housing.

In some embodiments, the intermediate and outer members are removable together from the housing.

There is provided a method of assembling the cap of the injection device comprising the following steps: holding the intermediate member and the inner member in an engaged position, relative to one another; clipping the outer member onto the inner member to form a sub assembly; installing the sub assembly on a housing assembly, the housing assembly including the housing and the needle shield to cover the needle; priming the injection device; and clipping the lid onto the inner member and engaging via a press fit connection with the outer member.

The intermediate member can include a first engagement portion and the outer member can comprise a secondary engagement portion; the method comprising clipping the outer member onto the inner member and the secondary engagement portion of the outer member engaging with the primary engagement portion of the intermediate member.

The outer member can comprise an ergonomic surface comprising a high friction material or formations to enhance the gripping characteristics of the outer member so the cap can be more easily removed by a user. A further advantage is that if the cap has any condensation on it from being stored at cold temperatures it can become slippery and difficult to grip, or if it is still cold can be uncomfortable for a user to grip, the ergonomic surface can address this.

These and other aspects will be apparent from and elucidated with reference to the embodiments described hereinafter.

### BRIEF DESCRIPTION OF THE DRAWINGS

Embodiments will now be described, by way of example only, with reference to the accompanying drawings, in which:
Fig. 1A is a schematic side view of an auto-injector with a cap attached to a housing of the auto-injector;
Fig. 1B is a schematic side view of the auto-injector of Fig. 1A, with the cap removed from the housing;
Fig. 2 is a close-up schematic cross-sectional side view of a needle shield of an auto-injector;
Fig. 3 is a close-up isometric view of a cap according to one aspect showing internal components of the cap;

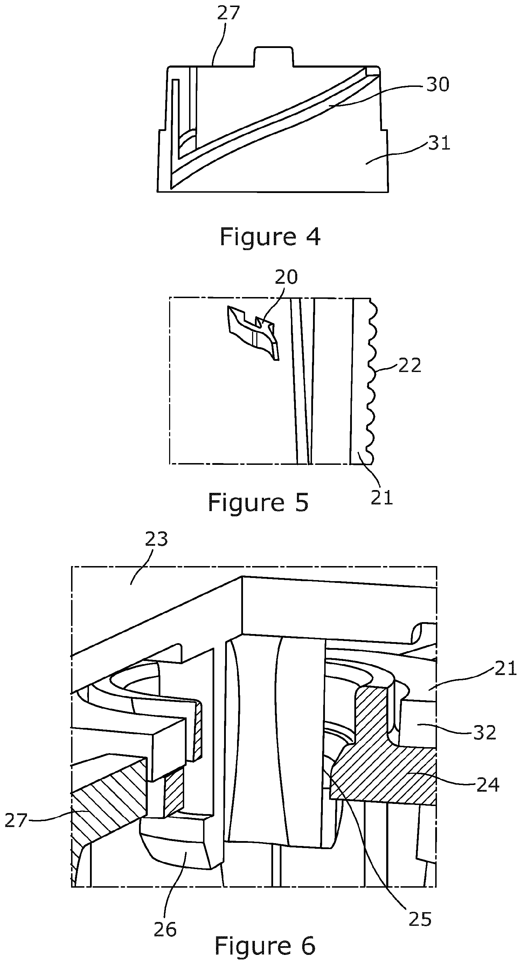

Fig. 4 is a close-up side view of the axially-inclined portion of the intermediate member of Fig. 3;
Fig. 5 is a close-up cross-sectional view of the outer member of Fig. 3 showing a lifting portion;
Fig. 6 is a close-up of section A of Figure 3;

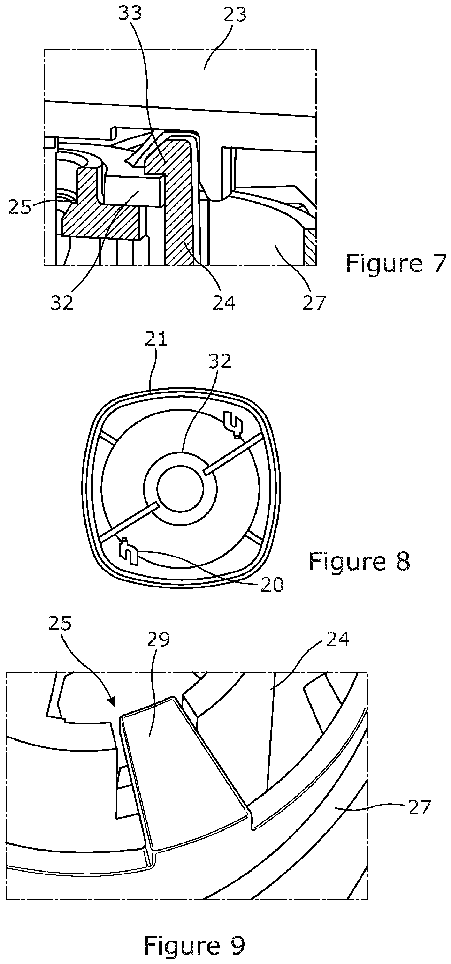

Fig. 7 is a close up view of an inner member clip of Fig. 3;
Fig. 8 is a plan view of the outer member of Fig. 3;
Fig. 9 is a close up isometric top view of the intermediate member and the inner member of Fig 3;

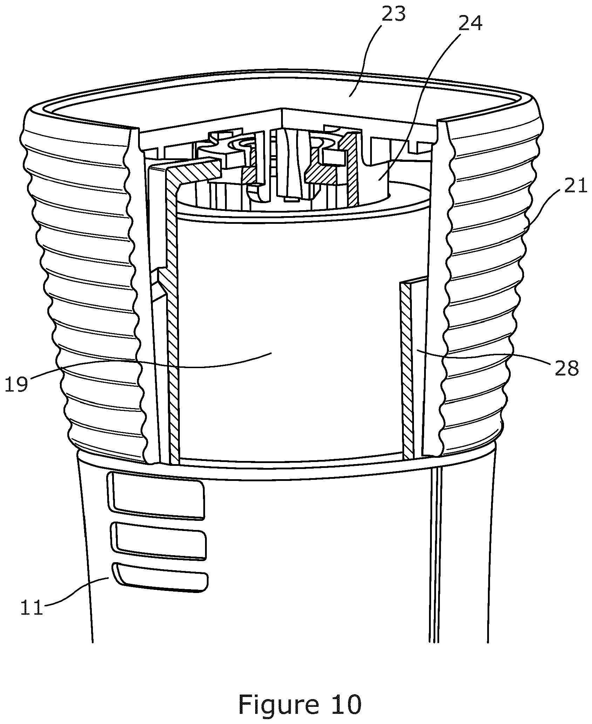

Fig. 10 is the cap of Fig.3 before it has been removed on an injection device such as those in Figs 1A and 1B;

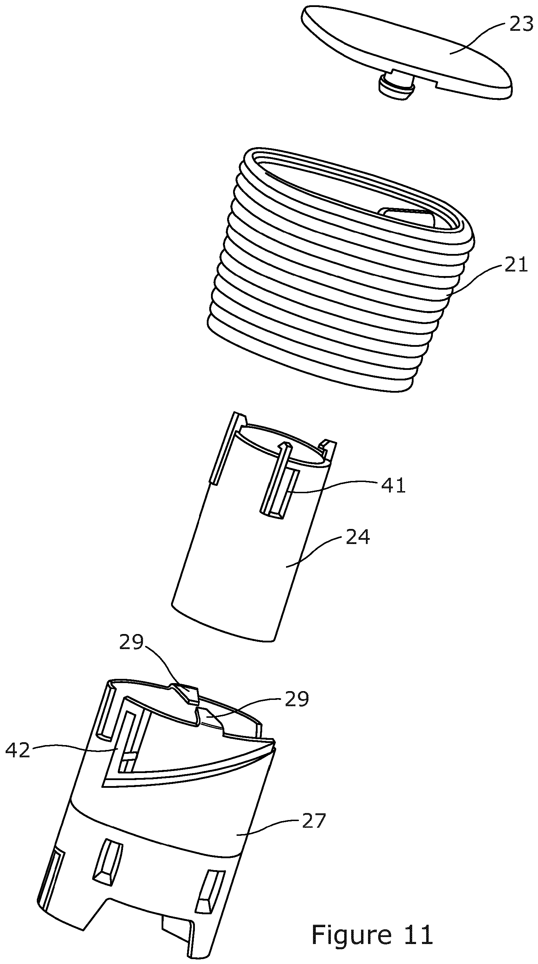

Fig. 11 is an exploded view of the main components of Figure 3;
Fig. 12 is a side view showing the outer member as transparent so internal components can be viewed at various stages of removing the cap of Fig 3;
Fig. 13 is a diagrammatic view of a cap according to a second aspect of the invention;
Fig. 14 is a cross-sectional side view of a cap according to a fourth aspect of the invention;
Fig. 15 is a close up view of the first lifting portion and axially-inclined portion of Fig 14 with the housing showed as transparent so internal components can be seen;
Fig. 16 is an alternative to Figure 15 with the axially-inclined portion on an external face of the housing where the axially-inclined portion is visible both before and after uncapping;
Fig. 17 is an alternative to Figure 15 with the axially-inclined portion on an external face of the housing where the axially-inclined portion is not visible before uncapping;

### DETAILED DESCRIPTION

A drug delivery device, as described herein, may be configured to inject a medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 2 ml. Yet another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 10 ml).

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 27 and 29 Gauge.

The delivery devices described herein can also include one or more automated functions. For example, one or more of needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may each be activated via an activation mechanism. Such an activation mechanism can include one or more of a button, a lever, a needle sleeve, or other activation component. Activation of an automated function may be a one-step or multi-step process. That is, a user may need to activate one or more activation components in order to cause the automated function. For example, in a one-step process, a user may depress a needle sleeve against their body in order to cause injection of a medicament. Other devices may require a multi-step activation of an automated function. For example, a user may be required to depress a button and retract a needle shield in order to cause injection.

In addition, activation of one automated function may activate one or more subsequent automated functions, thereby forming an activation sequence. For example, activation of a first automated function may activate at least two of needle insertion, medicament injection, and needle retraction. Some devices may also require a specific sequence of steps to cause the one or more automated functions to occur. Other devices may operate with a sequence of independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

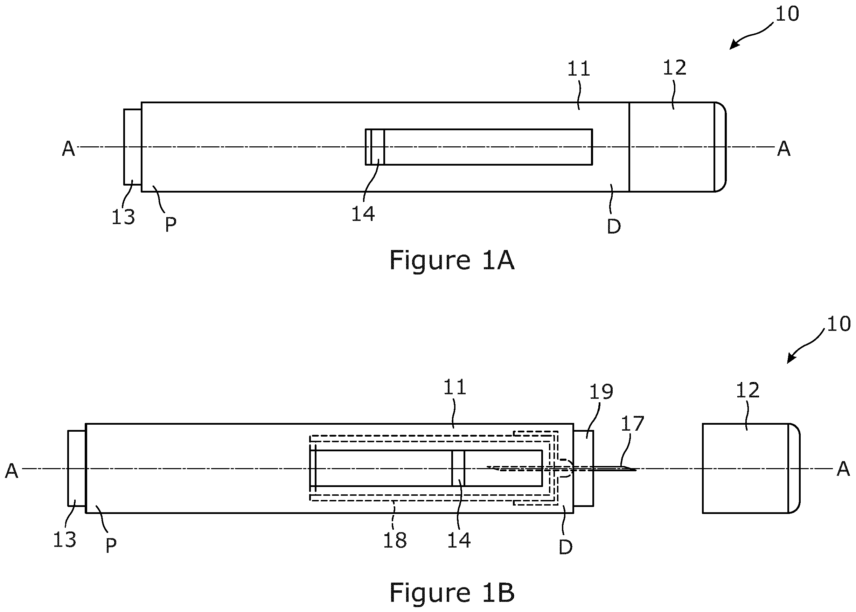

According to some embodiments of the present disclosure, an exemplary drug delivery device 10 is shown in Figs. 1A & 1B. Device 10, as described above, is configured to inject a medicament into a patient's body. Device 10 includes a housing 11 which typically contains a reservoir containing the medicament to be injected (e.g. a syringe) and the components required to facilitate one or more steps of the delivery process. Device 10 can also include a cap assembly or cap 12 that can be detachably mounted to the housing 11. Typically a user must remove cap 12 from housing 11 before device 10 can be operated.

As shown, housing 11 is substantially cylindrical and has a substantially constant diameter along the longitudinal axis A-A. The housing 11 has a distal region D and a proximal region P. The term "distal" refers to a location that is relatively closer to a site of injection, and the term "proximal" refers to a location that is relatively further away from the injection site.

Device 10 can also include a needle sleeve 19 coupled to housing 11 to permit movement of sleeve 19 relative to housing 11. For example, sleeve 19 can move in a longitudinal direction parallel to longitudinal axis A-A. Specifically, movement of sleeve 19 in a proximal direction can permit a needle 17 to extend from distal region D of housing 11.

Insertion of needle 17 can occur via several mechanisms. For example, needle 17 may be fixedly located relative to housing 11 and initially be located within an extended needle sleeve 19. Proximal movement of sleeve 19 by placing a distal end of sleeve 19 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 19.

Another form of insertion is "automated," whereby needle 17 moves relative to housing 11. Such insertion can be triggered by movement of sleeve 19 or by another form of activation, such as, for example, a button 13. As shown in Figs. 1A & 1B, button 13 is located at a proximal end of housing 11. However, in other embodiments, button 13 could be located on a side of housing 11.

Other manual or automated features can include drug injection or needle retraction, or both. Injection is the process by which a bung or piston 14 is moved from a proximal location within a syringe 18 to a more distal location within the syringe 18 in order to force a medicament from the syringe 18 through needle 17. In some embodiments, a drive spring (not shown) is under compression before device 10 is activated. A proximal end of the drive spring can be fixed within proximal region P of housing 11, and a distal end of the drive spring can be configured to apply a compressive force to a proximal surface of piston 14. Following activation, at least part of the energy stored in the drive spring can be applied to the proximal surface of piston 14. This compressive force can act on piston 14 to move it in a distal direction. Such distal movement acts to compress the liquid medicament within the syringe 18, forcing it out of needle 17.

Following injection, needle 17 can be retracted within sleeve 19 or housing 11. Retraction can occur when sleeve 19 moves distally as a user removes device 10 from a patient's body. This can occur as needle 17 remains fixedly located relative to housing 11. Once a distal end of sleeve 19 has moved past a distal end of needle 17, and needle 17 is covered, sleeve 19 can be locked. Such locking can include locking any proximal movement of sleeve 19 relative to housing 11.

Another form of needle retraction can occur if needle 17 is moved relative to housing 11. Such movement can occur if the syringe 18 within housing 11 is moved in a proximal direction relative to housing 11. This proximal movement can be achieved by using a retraction spring (not shown), located in distal region D. A compressed retraction spring, when activated, can supply sufficient force to the syringe 18 to move it in a proximal direction. Following sufficient retraction, any relative movement between needle 17 and housing 11 can be locked with a locking mechanism. In addition, button 13 or other components of device 10 can be locked as required.

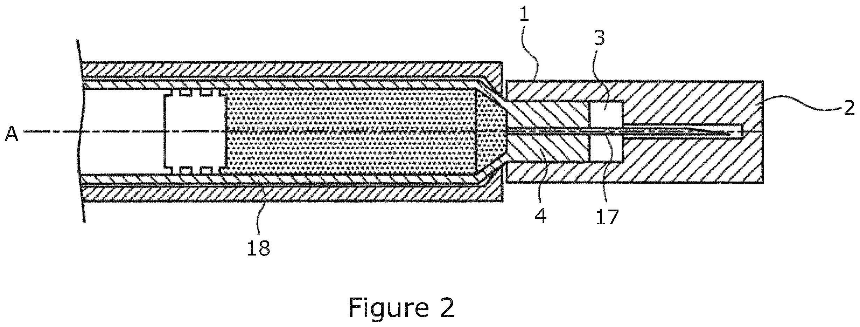

Referring now to Fig. 2 an injection device 10 can further comprise a needle shield 1. The needle shield 1 comprises a body 2 of impermeable material with a recess 3 in the proximal end of the body 2. The recess 3 is configured to receive the needle hub 4 and the needle 17 such that the needle 17 is shielded by the body 2. The inside surface of the body 2 and the outside surface of the needle hub 4 frictionally engage to seal the recess 3 to prevent the ingress of air into the recess 3. Thus, the needle 17 is kept sterile when the cap 12 is attached to the housing 11.

It can be advantageous for the cap 12 and the needle shield 1 to be removed simultaneously which makes removal of the cap 12 and needle shield 1 easier for a user. The force required to remove the cap 12 from the injection device 10 can be relatively high such that an elderly or physically impaired user may find removing the cap difficult. One reason this difficulty can arise is due to the frictional engagement between the needle shield 1 and the needle hub 4. However, it can be appreciated that the force required varies based on the materials of construction, the design of the device and the ambient temperature among other variables. Furthermore, the force required to remove the needle shield and the cap can be increased further by cold temperatures. For some medicaments and therefore some injection devices there is a requirement or need to store the device in the fridge or at low temperatures. This can exacerbate the difficulty in removing the cap 12 from the housing 11.

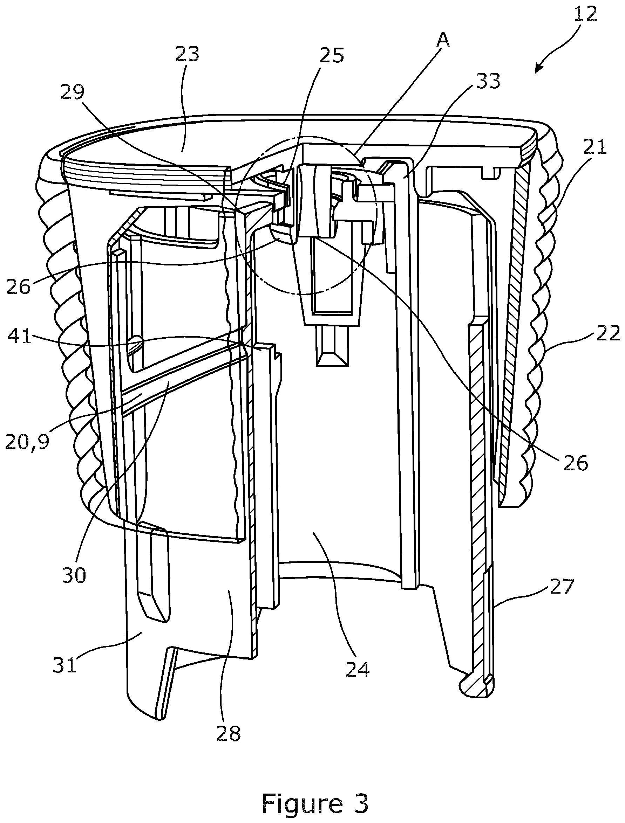

Illustrated in Figure 3, there is a removable cap 12 having an outer member 21 with formations 22 to enhance the gripping characteristics of the outer member 21. It can be appreciated however that the outer member 21 may be made of a higher friction material, it may comprise a further coating, it may be specifically shaped ergonomically, or it may have no friction enhancing properties. The outer member 21 is coupled to a lid 23, the lid can be coupled to the outer member 21 by a press fit connection or alternative known connection means, the outer member 21 and the lid 23 can also be integrally formed so they are one component as they are fixed both axially and rotationally to one another. The outer member 21 comprises a lifting portion 20 which in this instance is an inwardly protruding boss 9. The outer member 21 further comprises an annulus 32.

The removable cap 12 has an inner member 24 with an aperture 25, the lid 23 has clips 26 which engage with the inner member 24 via the central aperture 25. This engagement axially couples the lid 23 to the inner member 24, however allows the lid 23 to rotate with respect to the inner member 24. The inner member 24 further comprises clips 33 which clip onto the annulus 32 of the outer member 21. This axially couples the outer member 21 and inner member 24 but allows them to rotate in relation to one another. The inner member 24 can interface or be configured to engage with the needle shield 1, this can be in the form of but not limited to, grippers, a barb cutting into or sitting tightly against the needle shield 1 or a frictional lock between the inner member 24 and the needle shield 1. The inner member 24 being configured to engage with the needle shield can mean that there is a separate component such as a grabber (not shown) attached or located within the inner member 24 which can further comprise any of the mechanisms described above.

The cap 12 also comprises an intermediate member 27 which is located substantially between the inner member 24 and the outer member 21. The intermediate member 27 has a substantially tubular body 28 with at least one inwardly projecting formation 29 at its distal end. The formation 29 is for engagement with a cut out 41 of the inner member 24, the inwardly projecting formation 29 and cut out 41 providing a stop mechanism. The inner member 24 is free to move axially in a distal direction away from the housing with respect to the intermediate member 27, until a first stop portion (i.e. the inwardly projecting formation 29) of the intermediate member 27 is engaged with a second stop portion (i.e. the proximal most extremity of cut out 41). When the first stop portion is engaged with the second stop portion the inner member 24 is fixed axially to the intermediate member 27. The stop mechanism also rotationally couples the intermediate member 27 to the inner member 24. An advantage of this feature is that the intermediate member 27 is not fixed axially to the inner member 24 until the stop portions are engaged. This enables the inner member 24 to move the needle shield 1 in a distal direction independently of the intermediate member 27 until the stop portions engage, the inner member 24 and intermediate member 27 are then fixed axially for the final lift of the cap from the housing. It can be appreciated that the first stop portion could be a cut out, and the second stop portion could be a projection; furthermore the skilled person can envisage a number of alternative known means for the first and second stop portions which would provide a stopping mechanism as described above.

The intermediate member 27 also comprises an axially inclined portion 30 which is in the form of a ramped groove in an outer wall 31 of the intermediate member. The ramp can be an indent in the outer wall 31, or a cut out the entire way through the intermediate member 27. The ramp is inclined; however it can be appreciated that it can incline in either direction, it may also be a curved shape or comprise more than one gradient. The inclination of the ramp required is dependent on the direction of the rotational movement of the outer member 21 required to remove the cap 12. The intermediate member 27 is also fixed rotationally to the housing 11 which prevents the intermediate member 27 and therefore inner member 24 from rotational movement even when the outer member 21 is twisted or rotated.

It can be appreciated that there a number of ways which would be known to a person skilled in the art to rotationally and axially couple two components to one another. Any combination of grooves, clips, bosses, formations and protrusions can be used to couple each component described above allowing either rotational movement, axial movement or neither depending on the requirements described above. It can also be appreciated that the lifting portion 20 may be located on the intermediate member 27 and the axially-inclined portion 30 may be located on the outer member 21.The first stop portion may be located on the inner member 24, the second stop portion located on the intermediate member 27. The stop portions can also be any known mechanism described above which allows relative axial movement to a point and then prevents relative axial movement past that point.

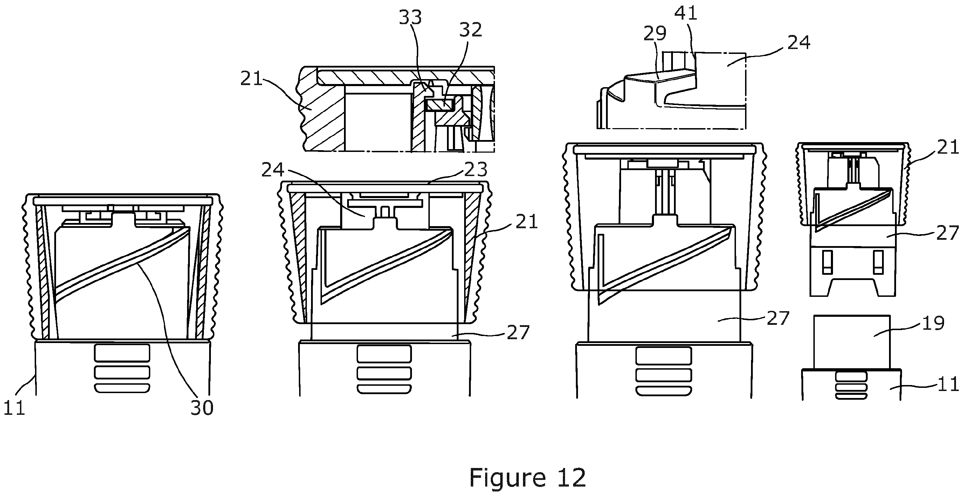

The mechanism of removal of the cap 12 from the housing 11 can be described as follows and can be seen in the steps shown in Figure 12. As a user twists or rotates the outer member 21, the force is transmitted from the outer member 21 to the inner member 24 through the clips 33. The inner member 24 is moved in a distal direction away from the housing 11. The lifting portion 20 travels along the axially-inclined portion 30 from a first position to a second position, i.e. the inwardly protruding boss travels along the ramp as the outer member 21 is twisted. The first position being a proximal most end of the axially inclined portion and the second position being a distal most end of the axially inclined portion. At the end of the ramp, the intermediate member 27 and outer member 21 are fixed axially to one another. The outer member 21 is then lifted, removing the cap with less force than would have been required before twisting of the outer member 21 and more smoothly due to the momentum buildup of the method described above. The injection device 10 may further comprise a needle shield 1 and removal of the cap 12 removes the needle shield 1 simultaneously.

A method of assembling the cap 12 of the injection device includes the following steps. The intermediate member 27 and the inner member 24 are held in position in the assembly equipment. The outer member 21 can then be clipped onto the inner member 24 forming a sub assembly. In some instances the intermediate member 27 incorporates a primary engagement portion which can be for example but not limited to, a thread, protrusion or a groove. The outer member 21 then comprises a secondary engagement portion which can be for example but not limited to a thread, protrusion or a groove to suit the primary engagement portion. The outer member 21 is clipped onto the inner member 24 the secondary engagement portion of the outer member 21 also engages with the primary engagement portion of the intermediate member 27. The sub assembly is installed on a housing assembly, the housing assembly having previously been assembled and including the housing 11 and the needle shield 1 to cover the needle 17. The injection device undergoes a priming action before the lid 23 is clipped onto the inner member 24 and engages via a press fit connection with the outer member 21.

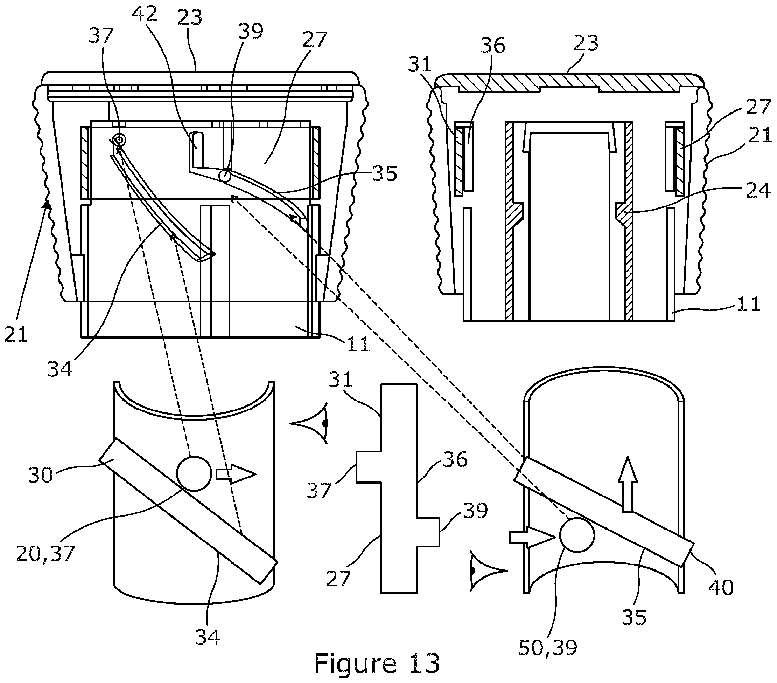

Another aspect of the invention is shown in Figure 13. A number of the features remain the same as in previous Figures and like components retain the same reference numerals. The intermediate member 27 has a first lifting portion 20 in the form of a primary protruding boss 37 and a second lifting portion 50 in the form of a secondary protruding boss 39. The outer member comprises a first axially-inclined portion 30 in the form of a primary ramp 34 and the inner member comprises a second axially-inclined portion 40 in the form of a secondary ramp 35. It can be appreciated that the ramps 34, 35 can be protruding from the inner or outer member, an indent or groove or a cut out the entire way through the inner or outer member. The ramp is inclined; however it can be appreciated that it can incline in either direction, curve, or comprise more than one gradient.

The primary protruding boss 37 is configured to engage with the primary ramp 34 from a first position to a second position, in the illustrated example from a distal most end of the ramp to a proximal most end of the ramp. The intermediate member 27 is fixed axially to the inner member 24 and not the outer member 21. As the outer member is pulled in a distal direction away from the housing, the intermediate member 27 resists the axial movement due to the engagement with the inner member 24. The intermediate member 27 is then forced to rotate due to the engagement of the primary ramp 34 and primary boss 37. As the outer member 21 is lifted and the intermediate member 27 rotates as described above, the secondary boss 39 is in engagement with the secondary ramp 35. The inner member is restricted from rotating with the intermediate member 27 as it is fixed rotationally to the housing 11 and therefore the engagement of the secondary boss 39 and the secondary ramp 35 causes the inner member to be forced up in a distal direction away from the housing 11. The cap 12 can then be removed from the housing.

When the first axially inclined portion 30 has a gradient shallower in an axial direction than the gradient of the second axially inclined portion 40 as shown in Figure 13 then the axial movement of the outer member 21 from pulling in a distal direction is greater than the axial of movement of the inner member. In this instance the axial force on the inner member 24 is greater than the axial force applied to the outer member 21. It can be appreciated that this can be the opposite way round and that the second axially inclined portion 40 could have a gradient shallower in an axial direction than the gradient of the first axially inclined portion. This would mean that as the outer member 21 is pulled from the housing 11 as described above, the axial movement of the outer member 21 causes an axial movement of the inner member which is greater than the axial movement of the outer member.

It can be appreciated that there a number of ways which would be known to a person skilled in the art to rotationally and axially couple two components to one another. Any combination of grooves, clips, bosses, formations and protrusions can be used to couple each component described above allowing either rotational movement, axial movement or neither depending on the requirements described above. It can also be appreciated that the lifting portions 20,50 may be located on the outer member and the inner member and the axially-inclined portions 30,40 may be located on the intermediate member 27 or any combination of the above. It can be considered that the outer member 21 and inner member 24 comprise the ramps and the intermediate member 27 comprises the protruding boss, one facing outwards towards the outer member and away from the central axis of the device, and another boss facing inwards towards the inner member and towards the central axis of the device, or any combination of the above which facilitates the mechanism described above.

The injection device 10 may comprise a holding portion 42 at one end of the axially-inclined portion 30 or the second axially-inclined portion 40 to engage with the lifting portion 20 or second lifting portion 50 to resist relative movement of the cap 12 and the housing 11 before use. It may be required that the cap is pushed in a proximal direction to release the lifting portion from the holding portion 42 before removal of the cap. An advantage of this is that premature removal of the cap is prevented, this feature may also prevent movement of particular components of the device during storage or transport which could damage the device.

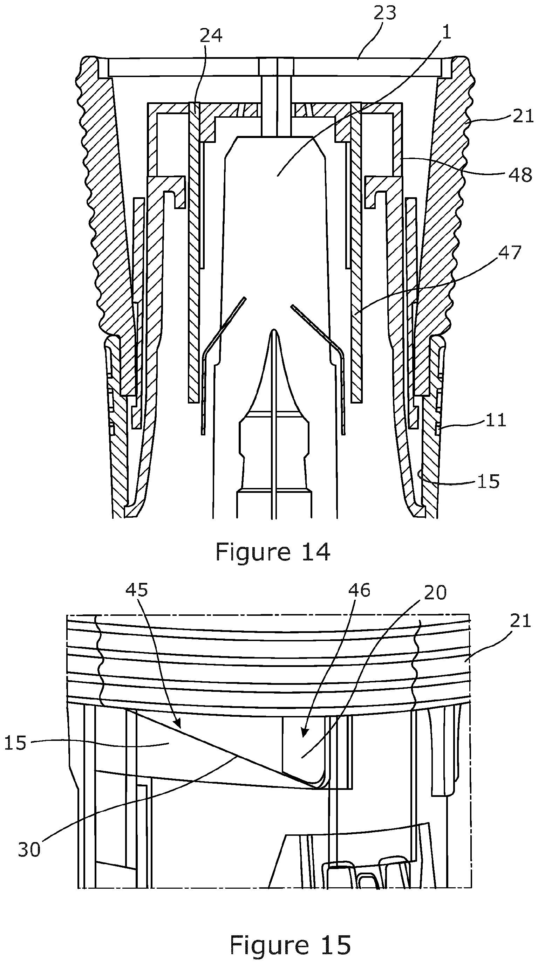

Another aspect of the invention is shown in Figures 14 and 15, a number of the features remain the same as previous Figures and like components retain the same reference numerals. In this aspect an inner wall 15 of the housing 11 comprises the axially-inclined portion 30, which in this aspect is an internal ramp 45. The lifting portion 20 being a proximally protruding boss 46, the boss 46 extends downwards from a proximal edge of the outer member 21 and in a proximal direction of the outer member 21. As the outer member is rotated the proximally protruding boss 46 engages with the internal ramp 45, lifting the outer member 21, the inner member 24 being fixed rotationally to the needle shield 1, but axially locked to the lid 23 and the outer member 21. Therefore the rotation of the outer member 21 and lid 23 allows the cap to be lifted and thus removed without rotation of the needle shield 1, needle sleeve or inner member 24. It can be appreciated that the inner member 24 can be made of two parts, an inner tube 47 and an outer tube 48. If coring, as described below is not an issue then the inner tube 47 can be integral or fixedly coupled to the outer member or lid. Furthermore it can be envisaged that the outer tube 48 may be rotatable with respect to the inner tube 47.

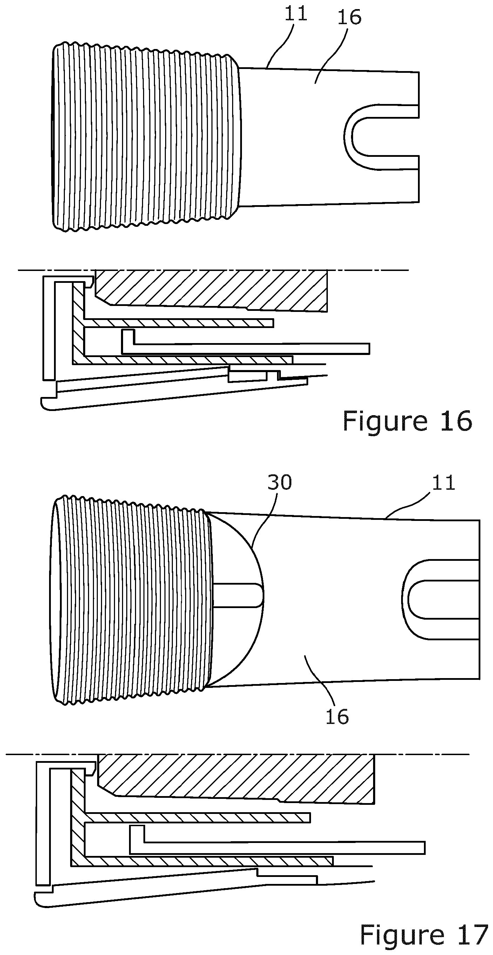

It can be appreciated that the axially-inclined portion 30 described above can be disposed on an external face 16 of the housing as shown in Figure 17, this enables the axially-inclined portion 30 to be visible both before and after uncapping (removing the cap from the injection device). Furthermore the axially-inclined portion 30 can be on the external face 16 of the housing however hidden by a longer length outer member 21 as in Figure 16 extending further in a proximal direction than in other aspects. Wherein the axially-inclined portion 30 is concealed before the cap is removed from the injection device, however visible after the cap has been removed from the injection device.

It can be appreciated that the axially-inclined portion 30 is half-moon shaped, semicircular, linear with an incline in either direction, V-shaped or an alternative ramped shape. Any shape can be envisaged that would suitably enable a lifting portion to engage with it and to cause an inner member to be lifted in a distal direction as described in any aspect above as the outer member 21 is rotated or lifted. The lifting portion may be a protrusion extending inwardly, outwardly, proximally or distally of the central axis of the housing depending on the corresponding axially-inclined portion. The lifting portion and axially-inclined portion may be disposed on an inner surface or outer surface of the housing or the outer member.

The lifting portion is a protrusion on the outer member extending axially in a proximal direction from a proximal edge of the outer member, the axially-inclined portion being a recess or protrusion on the housing;

The lifting portion can be a protrusion on the housing extending axially in a distal direction from a distal edge of the housing, the axially-inclined portion being a recess or protrusion on the outer member;

The lifting portion can be an inwardly protruding boss on an inner surface of the outer member, extending towards the central axis of the housing, the axially-inclined portion being a recess or protrusion on the housing; and/or

The lifting portion can be an outwardly protruding boss on an outer surface of the housing, extending away from the central axis of the housing, the axially-inclined portion being a recess or protrusion on the outer member.

Furthermore it can be appreciated that the lifting portion is on one of the housing 11 and the outer member 21 and the axially-inclined portion is on the other of the housing 11 and the outer member 21.

The lifting mechanism described above enables an interface between the outer member 21 and housing 11 which is non-circular in shape, for example but not limited to oval, rounded square or triangular shaped. This is due to the fact that the outer member 21 is lifted from the housing 11 as the outer member 21 rotates relative to the housing 11. This leaves the only contact between the outer member 21 and the housing 11 being the lifting mechanism.

It can be appreciated that any gradient or axially-inclined portion described in this specification could be also be non-constant. This would provide an advantage that the gearing the gradient provides can match the needle shield removal force which is also non-constant, i.e. in some embodiments as the cap is removed the force builds uniformly and then jumps to a peak. Therefore a variation in the gradients could provide gearing to provide low initial assistance to a user, followed by high assistance to a user to match the higher force required at the peak.

It can be appreciated that any lifting portion described in this specification can be for example but not limited to, a groove, clip, boss, formation, protrusion, cam or follower. It can be appreciated that any axially inclined portion described in this specification can be for example but not limited to a protrusion, an indent or groove or a cut out. The axially inclined portion is inclined; however it can be appreciated that it can incline in either direction, curve, or comprise more than one gradient. The lifting portion must be able to engage with the axially inclined portion to actuate axial movement from rotation, or actuate rotation from an axial movement.

It can be appreciated that the inner member of any embodiment described in this specification can be integral to the outer member or formed of two parts, an inner tube 47 and an outer tube 48, the inner tube 47 being integral to the outer member 21, where coring is not an issue. Coring is the damage of a needle 17 by rotation of the needle shield 1. The end of the needle 17 cuts a small portion of an inner surface of the needle shield 1 away as the needle shield 1 rotates with respect to the needle 17. As the needle shield 1 is then removed from the syringe 18 the cut portion can remain in the needle 17 blocking the needle 17 and damaging the injection device 10.

The injection device 1 may be disposable or it may be reusable.

The injection device 1 may provide a fixed dose or a user-settable dose.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia.

Examples of DPP4 inhibitors are Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which is defined by the appended claims.

## Claims

1. An injection device (10) comprising an elongate housing (11) having a central axis and a cap (12) provided at a distal end of the housing (11), the cap (12) being removable from the housing (11) and comprising:
an outer member (21) for a user to grip when removing the cap (12) from the housing (11);
an intermediate member (27) disposed substantially within the outer member (21), at least one of the intermediate member (27) or outer member (21) being rotatable relative to the other of the intermediate member (27) or outer member (21);
an inner member (24) disposed substantially within the intermediate member (27), and coupled to at least one of the outer member (21) and the intermediate member (27), the inner member (24) being configured to engage a needle shield (1) of a syringe (18) of medicament when such a syringe (18) is received in the housing (11), wherein the injection device (10) further comprises a lifting mechanism configured to reduce a force required to remove the cap (12) and an engaged needle shield (1) from the housing (11), the lifting mechanism comprising at least one axially-inclined portion (30, 40) in engagement with at least one lifting portion (20, 50) such that rotational movement of the outer member (21) or intermediate member (27) is converted into an axial movement of the inner member (24) in a distal direction from the housing (11).

2. An injection device (10) according to claim 1, wherein the outer member (21) includes a lid (23) and the inner member (24) comprises an aperture (25) at a distal end in engagement with a fixing portion (26) on the lid (23), such that the inner member (24) is coupled to the lid (23) so as to be fixed axially with respect to the lid (23) and to be rotatable with respect to the lid (23).

3. An injection device (10) according to claim 1 or claim 2, wherein the outer member (21) comprises an annulus shaped portion (32) in engagement with clips (33) on a distal end of the inner member (24) such that the inner member (24) is fixed axially with respect to the outer member (21) and rotatable with respect to the outer member (21).

4. An injection device (10) according to any preceding claim, wherein the intermediate member (27) and inner member (24) comprise a stop mechanism, the inner member (24) is free to move axially in a distal direction with respect to the intermediate member (27) until a first stop portion (29) of the intermediate member (27) engages with a second stop portion (41) of the inner member (24) such that the inner member (24) is prevented from further axial movement relative to the intermediate member (27).

5. An injection device (10) as claimed in any preceding claim, wherein: the axially-inclined portion (30) is a groove or cut out and the lifting portion (20) is a boss (9), the lifting portion (20) is located substantially within the axially-inclined portion (30), wherein as the outer member (21) is twisted the lifting portion (20) is forced to follow a path of the axially-inclined portion (30), causing axial movement of the outer member (21) and the inner member (24) in a distal direction; or, wherein the axially-inclined portion (30) is a ridge or projection (45) and the lifting portion (20) is a boss (46), the lifting portion (20) is axially offset from the axially-inclined portion (30) but in contact with a distal or proximal edge of the axially-inclined portion (30), wherein as the outer member (21) is twisted the lifting portion (20) is forced to follow a path of the axially-inclined portion (30) causing axial movement of the outer member (21) and the inner member (24) in a distal direction.

6. An injection device according to any of claims 1 to 4, wherein the axially-inclined portion (30) is a first axially-inclined portion (30) located on one of an outer face of the intermediate member (27) and an inner face of the outer member (21), and the lifting portion (20) is a first lifting portion (20) on the other of the outer face of the intermediate member (27) or the inner face of the outer member (21), the outer face being directed away from a central axis of the injection device (10) and the inner face being directed towards a central axis of the injection device (10) and, preferably, wherein the lifting mechanism comprises a second lifting portion (50) and a second axially-inclined portion (40), the second lifting portion (50) being located on one of an inner face of the intermediate member (27) and an outer face of the inner member (24) and the second axially-inclined portion (40) being on the other of the outer face of the inner member (24) and the inner face of the intermediate member (27), an outer face being directed away from a central axis of the injection device (10) and an inner face being directed towards a central axis of the injection device (10) and, preferably, wherein the second axially-inclined portion (40) has a shallower gradient with respect to the axial direction than the first axially-inclined portion (30).

7. An injection device (10) as claimed in claim 6, wherein at least one of the axially inclined portions (30, 40) is a groove or cut out and at least one of the lifting portions (20, 50) is a boss (37, 39), the at least one of the lifting portions (20, 50) is located substantially within the at least one of the axially-inclined portions (30, 40), wherein as the outer member (21) is lifted in a distal direction from the housing (11) each lifting portion (20, 50) is forced to follow a path of each axially-inclined portion (30, 40), causing rotational movement of the intermediate member (27) and axial movement of the inner member (24).

8. An injection device (10) as claimed in claim 6 or claim 7, wherein at least one of the axially-inclined portions (30, 40) is a ridge or projection (34, 35) and at least one of the lifting portions (20, 50) is a boss (37, 39), the at least one of the lifting portions (20, 50) is axially offset from the at least one of the axially-inclined portions (30, 40) but in contact with a distal or proximal edge of the at least one of the axially-inclined portions (30, 40), wherein as the outer member (21) is lifted in a distal direction away from the housing (11) each lifting portion (20, 50) is forced to follow a path of each axially-inclined portion (30, 40), causing rotational movement of the intermediate member (27) and axial movement of the inner member (24).

9. An injection device (10) according to any preceding claim comprising a holding portion (42) at one end of the axially-inclined portion (30) or the second axially-inclined portion (40) to engage with the lifting portion (20) or second lifting portion (50), wherein when the holding portion (42) and the lifting portion (20) or second lifting portion (50) are engaged the cap (12) and the housing (11) are held together and relative movement of the cap (12) and the housing (11) is resisted.

10. An injection device (10) according to any preceding claim, wherein the axially-inclined portion (30, 40) comprises more than one gradient to provide varying levels of assistance in removal of the cap (12) from the housing (11).

11. An injection device (10) according to any preceding claim, wherein the at least one axially-inclined portion (30) is in engagement with the at least one lifting portion (20) such that rotational movement of the outer member (21) or intermediate member (27) is converted into said axial movement of the inner member (24) in the distal direction from the housing (11) such that the axial movement of the inner member (24) in the distal direction from the housing (11) urges the engaged needle shield (1) in the distal direction from the housing (11).

12. An injection device (10) according to any preceding claim, wherein the inner member (24) is configured to engage said at least one of the outer member (21) and the intermediate member (27).

13. An injection device (10) according to any preceding claim, wherein the cap (12) comprises the lifting mechanism.

14. An injection device (10) as claimed in any preceding claim, further comprising a syringe (18) having a needle (17) disposed within the housing (11), wherein said syringe (18) contains a medicament.

15. A method of assembling the cap (12) to the injection device (10) of any of claims 2 to 14 comprising the following steps:
holding the intermediate member (27) and the inner member (24) in an engaged position, relative to one another;
clipping the outer member (21) onto the inner member (24) to form a sub assembly;
installing the sub assembly on a housing assembly, the housing assembly including the housing (11) and the needle shield (1) to cover the needle (17);
priming the injection device (10); and
clipping the lid (23) onto the inner member (24) and engaging via a press fit connection with the outer member (21).

## Patentansprüche

1. Injektionsvorrichtung (10), umfassend ein längliches Gehäuse (11), das eine mittlere Achse aufweist, und eine Kappe (12), die an einem distalen Ende des Gehäuses (11) bereitgestellt ist, wobei die Kappe (12) von dem Gehäuse (11) abnehmbar ist und Folgendes umfasst:
ein äußeres Element (21) zum Greifen für einen Benutzer beim Abnehmen der Kappe (12) von dem Gehäuse (11);
ein Zwischenelement (27), das im Wesentlichen innerhalb des äußeren Elements (21) angeordnet ist, wobei mindestens eines von dem Zwischenelement (27) oder äußeren Element (21) bezüglich des anderen von dem Zwischenelement (27) oder äußeren Element (21) drehbar ist;
ein inneres Element (24), das im Wesentlichen innerhalb des Zwischenelements (27) angeordnet und mit mindestens einem von dem äußeren Element (21) und dem Zwischenelement (27) gekoppelt ist, wobei das innere Element (24) konfiguriert ist, um einen Nadelschutz (1) einer Spritze (18) von Medikamenten einzugreifen, wenn eine solche Spritze (18) in dem Gehäuse (11) aufgenommen ist, wobei die Injektionsvorrichtung (10) ferner einen Hebemechanismus umfasst, der konfiguriert ist, um eine Kraft zu reduzieren, die erforderlich ist, um die Kappe (12) und einen eingegriffenen Nadelschutz (1) von dem Gehäuse (11) zu entfernen, der Hebemechanismus umfassend mindestens einen axial geneigten Abschnitt (30, 40) in Eingriff mit mindestens einem Hebeabschnitt (20, 50), sodass eine Drehbewegung des äußeren Elements (21) oder Zwischenelements (27) in eine axiale Bewegung des inneren Elements (24) in eine distale Richtung von dem Gehäuse (11) umgewandelt wird.

2. Injektionsvorrichtung (10) gemäß Anspruch 1, wobei das äußere Element (21) einen Deckel (23) beinhaltet und das innere Element (24) eine Öffnung (25) an einem distalen Ende in Eingriff mit einem Befestigungsabschnitt (26) an dem Deckel (23) umfasst, sodass das innere Element (24) mit dem Deckel (23) gekoppelt ist, um in Bezug auf den Deckel (23) axial fixiert und in Bezug auf den Deckel (23) drehbar zu sein.

3. Injektionsvorrichtung (10) gemäß Anspruch 1 oder Anspruch 2, wobei das äußere Element (21) einen ringförmig geformten Abschnitt (32) in Eingriff mit Clips (33) an einem distalen Ende des inneren Elements (24) umfasst, sodass das innere Element (24) in Bezug auf das äußere Element (21) axial fixiert und in Bezug auf das äußere Element (21) drehbar ist.

4. Injektionsvorrichtung (10) gemäß einem vorhergehenden Anspruch, wobei das Zwischenelement (27) und das innere Element (24) einen Anschlagmechanismus umfassen, wobei das innere Element (24) frei ist, um sich in Bezug auf das Zwischenelement (27) axial in eine distale Richtung zu bewegen, bis ein erster Anschlagabschnitt (29) des Zwischenelements (27) mit einem zweiten Anschlagabschnitt (41) des inneren Elements (24) eingreift, sodass das innere Element (24) an einer weiteren axialen Bewegung bezüglich des Zwischenelements (27) gehindert wird.

5. Injektionsvorrichtung (10) nach einem vorhergehenden Anspruch, wobei: der axial geneigte Abschnitt (30) eine Nut oder ein Ausschnitt ist und der Hebeabschnitt (20) ein Ansatz (9) ist, sich der Hebeabschnitt (20) im Wesentlichen innerhalb des axial geneigten Abschnitts (30) befindet, wobei, wenn das äußere Element (21) verdreht wird, der Hebeabschnitt (20) gezwungen wird, einem Weg des axial geneigten Abschnitts (30) zu folgen, was eine axiale Bewegung des äußeren Elements (21) und des inneren Elements (24) in eine distale Richtung bewirkt; oder, wobei der axial geneigte Abschnitt (30) eine Rippe oder ein Vorsprung (45) ist und der Hebeabschnitt (20) ein Ansatz (46) ist, der Hebeabschnitt (20) axial von dem axial geneigten Abschnitt (30) versetzt ist, aber in Kontakt mit einem distalen oder proximalen Rand des axial geneigten Abschnitts (30) ist, wobei, wenn das äußere Element (21) verdreht wird, der Hebeabschnitt (20) gezwungen wird, einem Weg des axial geneigten Abschnitts (30) zu folgen, was eine axiale Bewegung des äußeren Elements (21) und des inneren Elements (24) in eine distale Richtung bewirkt.

6. Injektionsvorrichtung gemäß einem der Ansprüche 1 bis 4, wobei der axial geneigte Abschnitt (30) ein erster axial geneigter Abschnitt (30) ist, der sich entweder an einer äußeren Fläche des Zwischenelements (27) oder an einer inneren Fläche des äußeren Elements (21) befindet, und der Hebeabschnitt (20) ein erster Hebeabschnitt (20) auf der anderen von der äußeren Fläche des Zwischenelements (27) oder der inneren Fläche des äußeren Elements (21) ist, wobei die äußeren Fläche von einer mittleren Achse der Injektionsvorrichtung (10) weggerichtet ist und die innere Fläche zu einer mittleren Achse der Injektionsvorrichtung (10) gerichtet ist, und vorzugsweise wobei der Hebemechanismus einen zweiten Hebeabschnitt (50) und einen zweiten axial geneigten Abschnitt (40) umfasst, sich der zweite Hebeabschnitt (50) entweder auf einer inneren Fläche des Zwischenelements (27) oder einer äußeren Fläche des inneren Elements (24) befindet, und wobei der zweite axial geneigte Abschnitt (40) auf der anderen von der äußeren Fläche des inneren Elements (24) und der inneren Fläche des Zwischenelements (27) ist, eine äußere Fläche, die von einer mittleren Achse der Injektionsvorrichtung (10) weggerichtet ist, und eine innere Fläche, die zu einer mittleren Achse der Injektionsvorrichtung (10) gerichtet ist, und, vorzugsweise, wobei der zweite axial geneigte Abschnitt (40) eine geringere Steigung in Bezug auf die axiale Richtung aufweist als der erste axial geneigte Abschnitt (30).

7. Injektionsvorrichtung (10) nach Anspruch 6, wobei mindestens einer von den axial geneigten Abschnitten (30, 40) eine Nut oder ein Ausschnitt ist und mindestens einer von den Hebeabschnitten (20, 50) ein Ansatz (37, 39) ist, sich der mindestens eine der Hebeabschnitte (20, 50) im Wesentlichen innerhalb des mindestens einen von den axial geneigten Abschnitten (30, 40) befindet, wobei, wenn das äußere Element (21) von dem Gehäuse (11) in eine distale Richtung angehoben wird, jeder Hebeabschnitt (20, 50) gezwungen wird, einem Weg von jedem axial geneigten Abschnitt (30, 40) zu folgen, was eine Drehbewegung des Zwischenelements (27) und eine axiale Bewegung des inneren Elements (24) bewirkt.

8. Injektionsvorrichtung (10) nach Anspruch 6 oder Anspruch 7, wobei mindestens einer von den axial geneigten Abschnitten (30, 40) eine Rippe oder ein Vorsprung (34, 35) ist und mindestens einer von den Hebeabschnitten (20, 50) ein Ansatz (37, 39) ist, der mindestens eine der Hebeabschnitte (20, 50) axial von dem mindestens einen von den axial geneigten Abschnitten (30, 40) versetzt ist, aber in Kontakt mit einem distalen oder proximalen Rand des mindestens einen von den axial geneigten Abschnitten (30, 40) ist, wobei, wenn das äußere Element (21) weg von dem Gehäuse (11) in eine distale Richtung angehoben wird, jeder Hebeabschnitt (20, 50) gezwungen wird, einem Weg von jedem axial geneigten Abschnitt (30, 40) zu folgen, was eine Drehbewegung des Zwischenelements (27) und eine axiale Bewegung des inneren Elements (24) bewirkt.

9. Injektionsvorrichtung (10) gemäß einem vorhergehenden Anspruch, umfassend einen Halteabschnitt (42) an einem Ende des axial geneigten Abschnitts (30) oder des zweiten axial geneigten Abschnitts (40), um mit dem Hebeabschnitt (20) oder dem zweiten Hebeabschnitt (50) einzugreifen, wobei, wenn der Halteabschnitt (42) und der Hebeabschnitt (20) oder der zweite Hebeabschnitt (50) in Eingriff sind, die Kappe (12) und das Gehäuse (11) zusammengehalten werden und einer relativen Bewegung der Kappe (12) und des Gehäuses (11) widerstanden wird.

10. Injektionsvorrichtung (10) gemäß einem vorhergehenden Anspruch, wobei der axial geneigte Abschnitt (30, 40) mehr als eine Steigung umfasst, um unterschiedliche Unterstützungsgrade beim Entfernen der Kappe (12) von dem Gehäuse (11) bereitzustellen.

11. Injektionsvorrichtung (10) gemäß einem vorhergehenden Anspruch, wobei der mindestens eine axial geneigte Abschnitt (30) mit dem mindestens einen Hebeabschnitt (20) in Eingriff ist, sodass eine Drehbewegung des äußeren Elements (21) oder Zwischenelements (27) in die axiale Bewegung des inneren Elements (24) in die distale Richtung von dem Gehäuse (11) umgewandelt wird, sodass die axiale Bewegung des inneren Elements (24) in die distale Richtung von dem Gehäuse (11) den eingegriffenen Nadelschutz (1) in die distale Richtung von dem Gehäuse (11) drängt.

12. Injektionsvorrichtung (10) gemäß einem vorhergehenden Anspruch, wobei das innere Element (24) konfiguriert ist, um mindestens eines von dem äußeren Element (21) und dem Zwischenelement (27) einzugreifen.

13. Injektionsvorrichtung (10) gemäß einem vorhergehenden Anspruch, wobei die Kappe (12) den Hebemechanismus umfasst.

14. Injektionsvorrichtung (10) nach einem vorhergehenden Anspruch, ferner umfassend eine Spritze (18), die eine Nadel (17) aufweist, die innerhalb des Gehäuses (11) angeordnet ist, wobei die Spritze (18) ein Medikament enthält.

15. Verfahren zum Zusammenbauen der Kappe (12) mit der Injektionsvorrichtung (10) nach einem der Ansprüche 2 bis 14, umfassend die folgenden Schritte:
Halten des Zwischenelements (27) und des inneren Elements (24) in einer eingegriffenen Position bezüglich zueinander;
Einclipsen des äußeren Elements (21) auf das innere Element (24), um eine Unterbaugruppe zu bilden;
Installieren der Unterbaugruppe an einer Gehäusebaugruppe, wobei die Gehäusebaugruppe das Gehäuse (11) und den Nadelschutz (1) beinhaltet, um die Nadel (17) abzudecken;
Vorfüllen der Injektionsvorrichtung (10); und
Aufclipsen des Deckels (23) auf das innere Element (24) und Eingreifen mit dem äußeren Element (21) mittels einer Presspassung.

## Revendications

1. Dispositif d'injection (10) comprenant un logement allongé (11) comportant un axe central et un capuchon (12) se trouvant au niveau d'une extrémité distale du logement (11), le capuchon (12) étant amovible du logement (11) et comprenant :
un élément externe (21) qu'un utilisateur peut saisir lors du retrait du capuchon (12) du logement (11) ;
un élément intermédiaire (27) disposé sensiblement à l'intérieur de l'élément externe (21), au moins un élément parmi l'élément intermédiaire (27) ou l'élément externe (21) pouvant tourner par rapport à l'autre élément parmi l'élément intermédiaire (27) ou l'élément externe (21) ;
un élément interne (24) disposé sensiblement à l'intérieur de l'élément intermédiaire (27) et couplé à au moins un élément parmi l'élément externe (21) et l'élément intermédiaire (27), l'élément interne (24) étant conçu pour venir en prise avec un protecteur d'aiguille (1) d'une seringue (18) de médicament lorsque cette seringue (18) est reçue dans le logement (11), ledit dispositif d'injection (10) comprenant en outre un mécanisme de levage conçu pour réduire la force requise pour retirer le capuchon (12) et un protecteur d'aiguille (1) en prise du logement (11), ledit mécanisme de levage comprenant au moins une partie axialement inclinée (30, 40) en prise avec au moins une partie de levage (20, 50) de sorte qu'un mouvement de rotation de l'élément externe (21) ou de l'élément intermédiaire (27) soit converti en un déplacement axial de l'élément interne (24) suivant une direction distale à partir du logement (11).

2. Dispositif d'injection (10) selon la revendication 1, ledit élément externe (21) comprenant un couvercle (23) et ledit élément interne (24) comprenant une ouverture (25) au niveau d'une extrémité distale en prise avec une partie de fixation (26) sur le couvercle (23), de sorte que l'élément interne (24) soit couplé au couvercle (23) de manière à être fixé axialement par rapport au couvercle (23) et à pouvoir tourner par rapport au couvercle (23).

3. Dispositif d'injection (10) selon la revendication 1 ou la revendication 2, ledit élément externe (21) comprenant une partie en forme d'anneau (32) en prise avec des clips (33) sur une extrémité distale de l'élément interne (24) de sorte que l'élément interne (24) soit fixé axialement par rapport à l'élément externe (21) et puisse tourner par rapport à l'élément externe (21).

4. Dispositif d'injection (10) selon l'une quelconque des revendications précédentes, ledit élément intermédiaire (27) et ledit élément interne (24) comprenant un mécanisme de butée, ledit élément interne (24) étant libre de se déplacer axialement suivant une direction distale par rapport à l'élément intermédiaire (27) jusqu'à ce qu'une première partie de butée (29) de l'élément intermédiaire (27) vienne en prise avec une seconde partie de butée (41) de l'élément interne (24) de sorte que l'élément interne (24) soit empêché de poursuivre son déplacement axial par rapport à l'élément intermédiaire (27).

5. Dispositif d'injection (10) selon l'une quelconque des revendications précédentes : ladite partie inclinée axialement (30) étant une rainure ou une découpe et ladite partie de levage (20) étant un bossage (9), ladite partie de levage (20) étant située sensiblement à l'intérieur de la partie inclinée axialement (30), lorsque l'élément externe (21) est tordu ladite partie de levage (20) étant contrainte de suivre le trajet de la partie inclinée axialement (30), ce qui provoque un déplacement axial de l'élément externe (21) et de l'élément interne (24) suivant une direction distale ; ou ladite partie inclinée axialement (30) étant une arête ou une saillie (45) et ladite partie de levage (20) étant un bossage (46), ladite partie de levage (20) étant décalée axialement par rapport à la partie inclinée axialement (30) mais en contact avec un bord distal ou proximal de la partie inclinée axialement (30), lorsque l'élément externe (21) est tordu ladite partie de levage (20) étant contrainte de suivre un trajet de la partie inclinée axialement (30), ce qui provoque un déplacement axial de l'élément externe (21) et de l'élément interne (24) suivant une direction distale.

6. Dispositif d'injection selon l'une quelconque des revendications 1 à 4, ladite partie inclinée axialement (30) étant une première partie inclinée axialement (30) située sur une face parmi une face externe de l'élément intermédiaire (27) et une face interne de l'élément externe (21), et ladite partie de levage (20) étant une première partie de levage (20) sur l'autre face parmi la face externe de l'élément intermédiaire (27) ou la face interne de l'élément externe (21), ladite face externe étant dirigée à l'opposé d'un axe central du dispositif d'injection (10) et ladite face interne étant dirigée vers un axe central du dispositif d'injection (10) et, de préférence, ledit mécanisme de levage comprenant une seconde partie de levage (50) et une seconde partie inclinée axialement (40), ladite seconde partie de levage (50) étant située sur une face parmi une face interne de l'élément intermédiaire (27) et une face externe de l'élément interne (24) et ladite seconde partie inclinée axialement (40) se trouvant sur l'autre face parmi la face externe de l'élément interne (24) et la face interne de l'élément intermédiaire (27), une face externe étant dirigée à l'opposé d'un axe central du dispositif d'injection (10) et une face interne étant dirigée vers un axe central du dispositif d'injection (10) et, de préférence, ladite seconde partie inclinée axialement (40) comportant une inclinaison plus faible par rapport à la direction axiale que la première partie inclinée axialement (30).

7. Dispositif d'injection (10) selon la revendication 6, au moins l'une des parties inclinées axialement (30, 40) étant une rainure ou une découpe et au moins l'une des parties de levage (20, 50) étant un bossage (37, 39), ladite au moins l'une des parties de levage (20, 50) étant située sensiblement à l'intérieur de ladite au moins l'une des parties inclinées axialement (30, 40), lorsque l'élément externe (21) est soulevé suivant une direction distale depuis le logement (11) chaque partie de levage (20, 50) étant contrainte de suivre un trajet de chaque partie inclinée axialement (30, 40), ce qui provoque un mouvement de rotation de l'élément intermédiaire (27) et un déplacement axial de l'élément interne (24).

8. Dispositif d'injection (10) selon la revendication 6 ou la revendication 7, au moins l'une des parties inclinées axialement (30, 40) étant une arête ou une saillie (34, 35) et au moins l'une des parties de levage (20, 50) étant un bossage (37, 39), ladite au moins l'une des parties de levage (20, 50) étant décalée axialement par rapport à ladite au moins l'une des parties inclinées axialement (30, 40) mais en contact avec un bord distal ou proximal de ladite au moins l'une des parties inclinées axialement (30, 40), lorsque l'élément externe (21) est soulevé suivant une direction distale à l'opposé du logement (11) chaque partie de levage (20, 50) étant contrainte de suivre un trajet de chaque partie inclinée axialement (30, 40), ce qui provoque un mouvement de rotation de l'élément intermédiaire (27) et un déplacement axial de l'élément interne (24).

9. Dispositif d'injection (10) selon l'une quelconque des revendications précédentes, comprenant une partie de maintien (42) au niveau d'une extrémité de la partie inclinée axialement (30) ou de la seconde partie inclinée axialement (40) pour venir en prise avec la partie de levage (20) ou la seconde partie de levage (50), lorsque la partie de maintien (42) et la partie de levage (20) ou la seconde partie de levage (50) sont en prise ledit capuchon (12) et ledit logement (11) étant maintenus ensemble et le mouvement relatif du capuchon (12) et du logement (11) rencontrant une résistance.

10. Dispositif d'injection (10) selon l'une quelconque des revendications précédentes, ladite partie inclinée axialement (30, 40) comprenant plus d'une pente pour fournir des niveaux variables d'assistance lors du retrait du capuchon (12) du logement (11).

11. Dispositif d'injection (10) selon l'une quelconque des revendications précédentes, ladite au moins une partie inclinée axialement (30) étant en prise avec ladite au moins une partie de levage (20) de sorte qu'un mouvement de rotation de l'élément externe (21) ou de l'élément intermédiaire (27) soit converti en ledit déplacement axial de l'élément interne (24) suivant la direction distale à partir du logement (11) de sorte que le déplacement axial de l'élément interne (24) suivant la direction distale à partir du logement (11) amène le protecteur d'aiguille (1) en prise suivant la direction distale à partir du logement (11).

12. Dispositif d'injection (10) selon l'une quelconque des revendications précédentes, ledit élément interne (24) étant conçu pour venir en prise avec ledit au moins un élément parmi l'élément externe (21) et l'élément intermédiaire (27).

13. Dispositif d'injection (10) selon l'une quelconque des revendications précédentes, ledit capuchon (12) comprenant le mécanisme de levage.

14. Dispositif d'injection (10) selon l'une quelconque des revendications précédentes, comprenant en outre une seringue (18) comportant une aiguille (17) disposée à l'intérieur du logement (11), ladite seringue (18) contenant un médicament.

15. Procédé d'assemblage du capuchon (12) au dispositif d'injection (10) selon l'une quelconque des revendications 2 à 14 comprenant les étapes suivantes :
le maintien de l'élément intermédiaire (27) et de l'élément interne (24) dans une position en prise l'un par rapport à l'autre ;
le clipsage de l'élément externe (21) sur l'élément interne (24) pour former un sous-ensemble ;
l'installation du sous-ensemble sur un ensemble logement, l'ensemble logement comprenant le logement (11) et le protecteur d'aiguille (1) pour recouvrir l'aiguille (17) ;
l'amorçage du dispositif d'injection (10) ; et
le clipsage du couvercle (23) sur l'élément interne (24) et la mise en prise par l'intermédiaire d'une connexion à ajustement par pression avec l'élément externe (21).
